# EUROPEAN PATENT APPLICATION

(11) **EP 2 932 913 A1**
(43) Date of publication of application: **21.10.2015**
(21) Application number: 14199650.4
(22) Date of filing: 22.12.2014
(51) Int. Cl.: A61B 17/072, A61B 17/29

(54) **End of life transmission system for surgical instruments**

(30) Priority: 17.04.2014 US 201461980824 P; 07.11.2014 US 201414535427
(71) Applicant: Covidien LP, Mansfield, MA 02048 (US)
(72) Inventor: Fitzsimmons, Thomas, New Milford, CT Connecticut 06776 (US); Grasso, Michelle, Shelton, CT Connecticut 06484 (US)
(74) Representative: Soames, Candida Jane

(57) **Abstract**

A powered handle (30) for a surgical instrument (10) includes a processor (22) and a component (110-180). The component has a usage limit and is configured to transmit a signature to the processor when the component is in proximity to its usage limit. The signature is configured to provide indicia directly to a user that the component is in proximity to its usage limit before the handle is used.

## Description

### CROSS-REFERENCE TO RELATED APPLICATIONS

This application claims the benefit of and priority to U.S. Provisional Patent Application No. 61/980,824, filed April 17, 2014, the entire disclosure of which is incorporated by reference herein.

### BACKGROUND

### 1. Technical Field

The present disclosure relates to surgical instruments and, more specifically, surgical instruments that transmit an end of life signature to a user.

### 2. Discussion of Related Art

A powered portion of a surgical instrument, typically a powered handle, is a complex and costly component that may be reused multiple times in a single surgical procedure. In addition, the powered portion of the surgical instrument may be sterilized after use and reused in subsequent surgical procedures.

Typically, the end of life of a powered surgical instrument is predetermined before the instrument is offered for sale. For example, the end of life for a powered surgical instrument may be determined during the engineering phase or the marketing phase of development of the surgical instrument. The end of life is generally represented by the number of cycles or uses of the powered surgical instrument.

Some components of a powered surgical instrument may fail before other components of the same powered surgical instrument depending on the type of use or the number of sterilizations of the powered surgical instrument. However, the predetermined end of life may not be able to account for the type of use or number of sterilizations of a particular surgical instrument. Accordingly, the predetermined end of life may remove or disable the powered surgical instrument prematurely (before it is approaching failure) or the powered surgical instrument may fail before reaching its predetermined end of life.

### SUMMARY

Accordingly, this disclosure relates to surgical instruments that monitor the actual use of each of the components of the surgical instrument. The surgical instruments provide indicia of an end of life based on the actual use of the components thereof. This monitoring may be used to extend the usable life of the surgical instrument and thus, reduce the cost of surgical procedures. In addition, the surgical instrument may detect a premature failure of a component within the surgical instrument and provide indicia to a user to replace the surgical instrument prior to its preset end of life. Further, the surgical instrument may communicate the premature failure to the manufacturer to allow premature failures to be analyzed and addressed as necessary in other existing surgical instruments or surgical instruments currently in development.

In an aspect of the present disclosure, a powered handle for a surgical instrument includes a processor and a component. The component has a usage limit and is configured to transmit a signature to the processor when the component is in proximity to its usage limit. The signature is configured to provide indicia directly to a user that the component is in proximity to its usage limit before the handle is used. The component may be one of a motor, a gear, or an electronic component. The indicia may be one of visual, audible, or haptic.

In aspects, the signature is acoustic and is configured to be audible to a user.

In some aspects, the signature may be configured to provide indicia of the remaining life of the component. The handle may include a display configured to provide indicia of the remaining life of the component.

In certain aspects, the handle includes a display configured to provide indicia to a user when the component is in proximity to its usage limit.

In particular aspects, the handle includes a sensor associated with a second component. The sensor is configured to detect when the second component is in proximity to is usage limit. The sensor may be configured to transmit a second signature to the processor when the second component is in proximity to its usage limit. The second signature may be configured to provide indicia directly to a user that the second component is in proximity to its usage limit before the handle is used.

In other aspects of the present disclosure, a powered surgical instrument includes an end effector, a handle, and a component having a usage limit. The handle is operatively associated with an end effector and includes a processor. The component is configured to transmit a signature to the processor when the component is in proximity to its usage limit. The signature is configured to provide indicia directly to a user that the component is in proximity to its usage limit before the handle is used.

In aspects, the component is disposed within the end effector.

In yet another aspect of the present disclosure, a method for using a surgical instrument includes activating a surgical instrument, gathering usage of a component of the surgical instrument, comparing usage of the component with a usage limit of the component, and transmitting a signature to a processor when the component is in proximity to its usage limit with the signature providing indicia directly to a user that the component is in proximity to its usage limit. The surgical instrument may be any of the surgical instruments disclosed herein and may include any of the handles disclosed herein. Comparing the usage of the component may include determining when the component is in proximity to its usage limit. The component may transmit the signature.

In aspects, gathering usage of the component includes a sensor that detects the usage of the component. The sensor may transmit the signature.

In some aspects, the method includes disabling the surgical instrument when the component is in proximity to its usage limit. The method may include displaying a remaining life of the component before using the surgical instrument.

In certain aspects, the method includes transmitting data including the usage of the component with a transmitter disposed within the surgical instrument to a processing unit that is remote to the surgical instrument. The processing unit may compare the usage of the component with a usage limit of the component to determine when the component is in proximity to its usage limit and may provide indicia to a sure when the component is in proximity to its usage limit.

In particular aspects, the method includes transmitting data including the usage of the component to a cloud with a transmitter disposed within the surgical instrument and analyzing the data at a location remote to the surgical instrument. The method may include accessing the data from the cloud and determining supply needs of the surgical instrument from the data.

Further, to the extent consistent, any of the aspects described herein may be used in conjunction with any or all of the other aspects described herein.

### BRIEF DESCRIPTION OF THE DRAWINGS

Various aspects of the present disclosure are described hereinbelow with reference to the drawings, wherein:
FIG. 1 is a schematic view of the components of a surgical system in accordance with the present disclosure illustrating a surgical instrument and a processing unit in perspective;
FIG. 2 is a cutaway view illustrating the internal components of the handle of the surgical instrument of FIG. 1;
FIG. 3 is a rear perspective view of the handle of the surgical instrument of FIG. 1;
FIG. 4 is another respective view of the handle of the surgical instrument of FIG. 1 illustrating the display showing an alert condition; and
FIG. 5 is a flow chart illustrating a method of using a surgical instrument in accordance with the present disclosure.

### DETAILED DESCRIPTION

Embodiments of the present disclosure are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" refers to the portion of the device or component thereof that is closest to the clinician and the term "distal" refers to the portion of the device or component thereof that is farthest from the clinician.

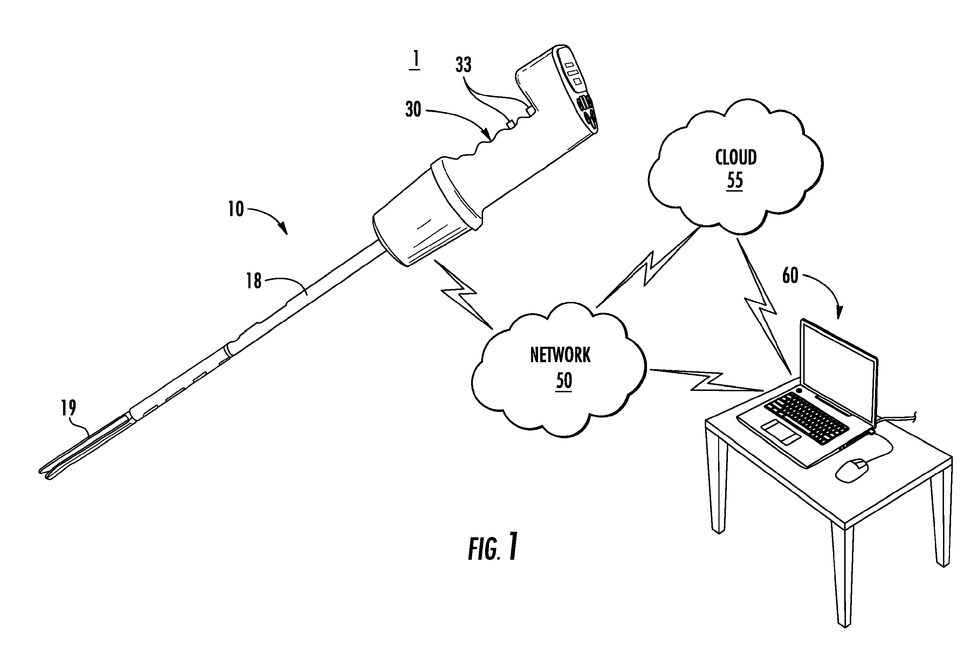

Referring now to FIG. 1, a surgical system 1 includes a powered surgical instrument 10 and a processing unit 60. The surgical instrument 10 may be in communication with the processing unit 60 via a network 50 or cloud 55 as detailed below. The surgical instrument 10 includes a powered handle 30, an adaptor 18 coupled to and extending from the handle 30, and an end effector 19 coupled to and extending from the adaptor 18. The end effector 19 is configured to act on tissue (e.g., drive staples into tissue, apply energy to tissue, cut tissue, apply a clip to tissue, manipulate tissue, etc.).

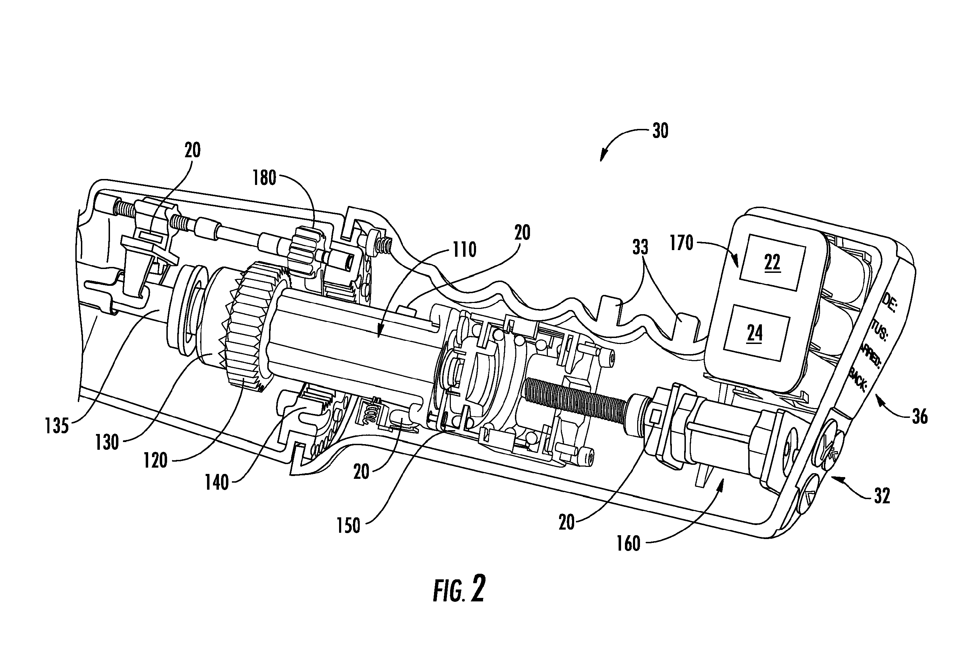

With reference to FIG. 2, the handle 30 includes components 110-180 disposed therein. The components 110-180 are configured to cooperatively manipulate the end effector 19 to act on tissue and/or to change the position of the end effector 19 relative to the handle 30. For example, when a drive motor 110 is activated, the drive motor 110 turns a drive gear 120 such that a driven gear 130 rotates a drive shaft 135 operatively associated with one or more functions of the end effector 19. Such a surgical instrument having a powered handle is disclosed in commonly owned and co-pending U.S. Patent Application Serial Nos. 11/894,959, filed on August 21, 2007 and published as U.S. Patent Publication No. 2008/0255413 on October 16, 2008; 13/331,047, filed December 20, 2011 and published as U.S. Patent Pub. No. 2012/0089131 on April 12, 2012; and 13/484,975, filed May 31, 2012 and published as U.S. Patent Pub. No. 2012/0253329 on October 4, 2012, the entire contents of each are incorporated by reference herein.

In embodiments, the components 110-180 of the surgical instrument 10 are in wired or wireless communication with a processor 22. The processor 22 is disposed within the handle 30. However, it is also within the scope of this disclosure that the processor 22 may be disposed anywhere within the surgical instrument 10 (e.g., the adaptor 18 or the end effector 19).

One or more of the components 110-180 of the handle 30 transmit actual usage of the component 110-180 to the processor 22 by a respective signature. The actual usage of the components 110-180 may include, but is not limited to, number of uses of the component, the temperature of the component, the force applied by the component, the force applied to the component, or damage to the component. Each signature may be an electronic or an acoustic signal. For example, non-limiting examples of signatures in accordance with the present disclosure include resonance of the components, capacitance of the components, inductance of the components, acoustic vibration of the components, etc.

The processor 22 analyzes the signatures to determine a real-time condition of the surgical instrument 10 including, but not limited to, the number of uses of the surgical instrument 10 remaining, if the surgical instrument 10 is approaching its end of life, if the surgical instrument 10 is beyond its end of life, etc. The processor 22 provides indicia of the condition of the surgical instrument 10 to a user. The processor 22 may provide visual, audible, or haptic feedback as indicia of the condition of the surgical instrument 10. It is also within the scope of this disclosure that when the signature is an acoustic signal, the signature, transmitted by the components 110-180, may be audible to a user to provide indicia as to the actual usage of the component or the condition of the surgical instrument 10.

The handle 30 may include sensors 20 disposed therewithin and in communication with the processor 22. Additionally or alternatively, sensors 20 may be disposed within the adaptor 18 or the end effector 19. Each sensor 20 may be an electrical contact, a proximity sensor, a strain gauge, an optical sensor, a photodiode, a mechanical or metallic sensor, or a combination thereof. Each sensor 20 monitors the use or the wear of one or more associated components 110-180.

Each sensor 20 may be in wired or wireless communication with the processor 22 to provide a signature to the processor 22. The signatures of the sensors 20 are substantially similar to the signatures of the components 110-180 as detailed above and will not be detailed herein for reasons of brevity.

Referring to FIGS. 1 and 2, the processor 22 transmits data, which may include the signatures, the actual usage of the components, or the condition of the surgical instrument 10 to a processing unit 60 remote to the surgical instrument 10 using a transmitter 24. The transmitter 24 may be integral to the processor 22. The transmission of data from the transmitter 24 to the processing unit 60 may be through a wired or wireless connection. The transmitter 24 may send data directly to the processing unit 60 or may send data to the processing unit 60 through a network 50. Additionally or alternatively, the transmitter 24 may send data to a cloud 55 and the processing unit 60 may retrieve the data from the cloud 55. It is also within the scope of this disclosure that one or more of the sensors 20 or the components 110-180 may send data to the cloud 55 or the processing unit 60 through a wireless connection. Additionally or alternatively, the processing unit 60 may transmit data to the processor 22. It is within the scope of this disclosure that the processing unit 60 receives a signature from one or more of the components 110-180.

Examples of the wireless connections described herein include, but are not limited to, radio frequency, optical, WIFI, Bluetooth^{®} (an open wireless protocol for exchanging data over short distances (using short length radio waves) from fixed and mobile devices, creating personal area networks (PANs)), ZigBee^{®} (a specification for a suite of high level communication protocols using small, low-power digital radios based on the IEEE 802.15.4-2003 standard for wireless personal area networks (WPANs)), etc. It is within the scope of this disclosure that the system may include a combination of different types of wireless connections.

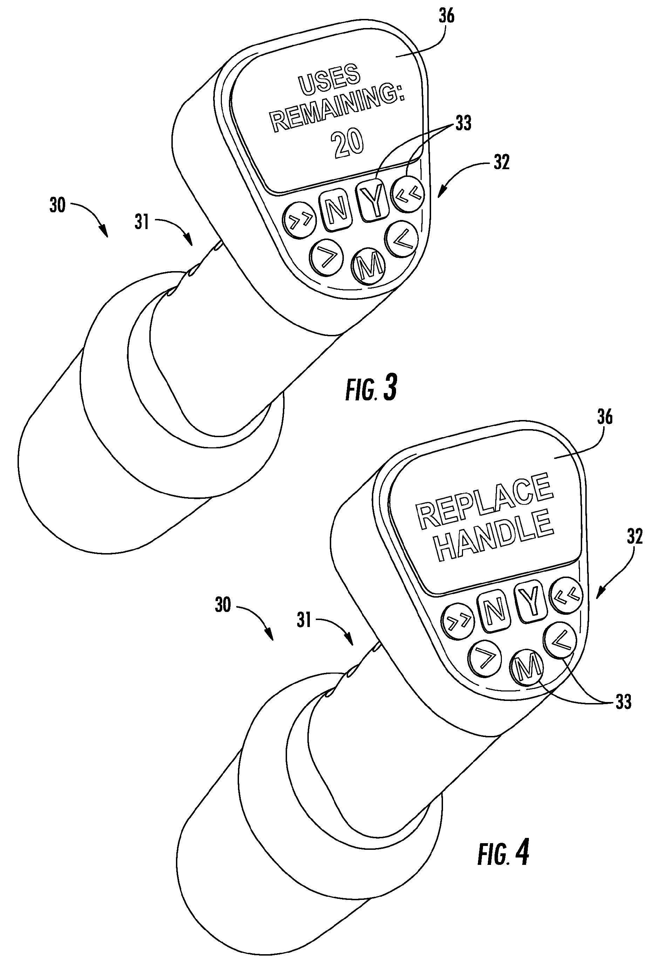

With additional reference to FIG. 3, the powered handle 30 includes a grip 31, a user interface 32, and a display 36. The grip 31 may be disposed substantially inline with the adaptor 18 or the end effector 19 as shown in FIG. 1 or may be substantially orthogonal to the adaptor 18 or the end effector 19 (e.g., a pistol grip). The user interface 32 includes control interfaces or buttons 33 to operate various functions of the surgical instrument 10 (e.g., opening and closing jaw members of the end effector 19, actuating the end effector 19 relative to the adaptor 18, rotating the end effector 19 about a longitudinal axis thereof, firing staples from the end effector 19, delivering electrosurgical energy with the end effector 19, etc.). The user interface 32 may include control interfaces 33 disposed on or adjacent to the grip 31 and/or the display 36. The control interfaces 33 may be levers, switches, buttons, touch screens, keyboards, digitizers, etc.

Continuing to refer to FIG. 3, the display 36 is positioned on the handle 30 such that the display is visible to a user during use of the handle 30. The display 36 displays indicia of the condition of the surgical instrument 10 including but not limited to a number of uses remaining (FIG. 3), an alert message (FIG. 4), the actual usage of the components 110-180 (e.g., the percentage of staples remaining in a cartridge (not shown), the angle of articulation of the end effector 19, the radial position of the end effector 19 about a longitudinal axis of the adaptor 18, a temperature of a component 110-180, etc.), environmental conditions about the surgical instrument 10 (e.g., the temperature adjacent the end effector 19, the temperature adjacent the handle 30, etc.).

The display 36 allows a user of the surgical instrument to proactively replace a surgical instrument 10 approaching or that will go beyond its end of life before the completion of a procedure. In addition, by monitoring the uses of the components (e.g., components 110-180) within the handle 30, a calculated end of life of the handle 30 may be determined based on the actual use of the handle 30. The actual use may include the number of cycles or uses of each component of the handle 20 (e.g., components 110-180) and/or the type of use of each of the components (e.g., the torque applied by or on a gear or by a motor, the duration of the use of a component) and/or an operating temperature of components 110-180. The calculated end of life may be greater than the predetermined end of life of the surgical instrument 10 as a whole enabling life of the surgical instrument 10 to be extended, and thus, reducing the overall cost of surgeries.

For example, a single component such as an articulation gear 180 may have a predetermined end of life of 50 uses. When usage limits were used to calculate the end of life of the surgical instrument 10, an average number of articulations per use may have been used to calculate the predetermined end of life of the surgical instrument. In this example, if it is determined that 1 out of every 6 uses is an articulation, then the predetermined end of life of the surgical instrument 10 would be 300 uses. However, if during actual use only one out of every 10 uses is an articulation, then the calculated end of life of the surgical instrument 10 based on the actual use of the articulation gear 180 would be 500 uses. Thus, the calculated end of life of the surgical instrument would be 200 uses greater than the predetermined end of life. Moreover, if the actual uses of the articulation gear 180 are greater than 1 in 6 uses (e.g., 1 in 5 or fewer uses), then the surgical instrument 10 may be removed from service before premature failure based on the actual use of the articulation gear 180. In addition, the actual uses of the articulation gear 180 relative to the other uses of the surgical instrument may be updated by the manufacturer by receiving global data from the cloud 55 of actual uses of the articulation gear 180 relative to other components of the surgical instrument 10. It will be appreciated that the calculated end of life may be adjusted in response to the actual uses of any component of the surgical instrument 10 in a manner similar to the example detailed above.

In some embodiments, the sensors 20 may detect failure or impending failure of an individual component 110-180 and send a signature to alert the user to replace the handle 30 or the surgical instrument 10. The sensor 20 may send an audible signature such as a tone or the sensor 20 may send an electronic signature to the processor 22 to provide visual indicia on the display 36 as shown in FIG. 4. In some embodiments, the processor 22 may disable the handle 30 from future use when the processor 22 receives a signature corresponding to failure of a component (e.g., components 110-180) of the handle 30 or the surgical instrument 10. Additionally or alternatively, the processor 22 may provide audible indicia of a failure or impending failure. In addition, the failure or impending failure may be transmitted as data to the cloud 55 (FIG. 1) or to the processing unit 60.

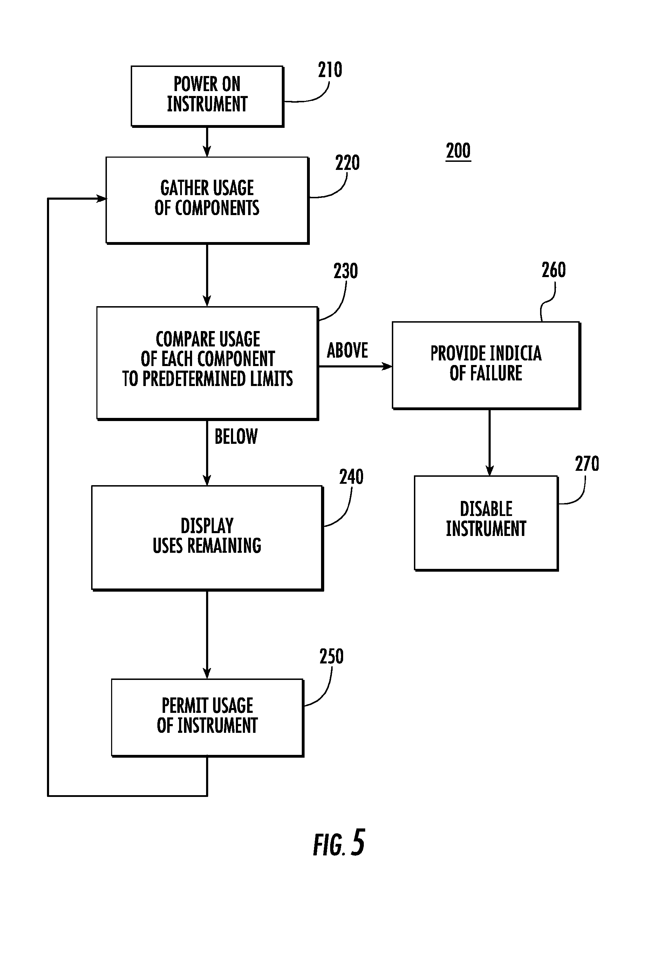

Referring to FIG. 5, a process 200 of permitting or disabling the surgical instrument 10 is set forth. It is contemplated that the processor 22 may permit or disable surgical instrument 10 using process 200. In addition, the processor 22 may calculate and provide the visual indicia of the remaining life of the surgical instrument 10. To begin, a user powers on or energizes surgical instrument 10 (Step 210). An energy source or battery 170 (FIG. 2) may be used to power the components of surgical instrument 10. The processor 22 gathers the usage of each of the components (e.g., components 110-180) of the surgical instrument 10 (Step 220). The usage may include number of actual uses or the number of actual uses with a particular condition (e.g., a rotation of a drive motor 110 with a torque over a limit). The processor 22 then compares the actual usage of each of the components with the usage limit for each of the individual components (Step 230).

If the actual usage of each of the components is below the usage limit for each component, the processor sends indicia to the user that the surgical instrument is ready to use (Step 240). The processor 22 may provide visual indicia to a user of a calculated number of uses of the surgical instrument 10 remaining before the surgical instrument reaches its calculated end of life on display 36. The calculated number of uses of the surgical instrument 10 may be the number of uses of the component with the fewest number of uses remaining before the component reaches its usage limit. The user may then use the surgical instrument 10 (step 250). After the use of the surgical instrument 10, the processor 22 repeats gathering the usage of each of the components of the surgical instrument 10 (Step 220).

If the actual usage of any of the components is over or beyond the usage limit for the respective component, the processor 22 then provides indicia of failure to the user via visual, audible, or haptic feedback (Step 260). For example, the processor 22 may send visual indicia, to replace the handle 30, to display 36, as shown in FIG. 4. In addition, the processor 22 may disable the handle 30 or instrument 10 (Step 270). Additionally or alternatively, the processor 22 may provide indicia that a replaceable component (not shown) of the surgical instrument 10 (e.g., a staple cartridge, a removable battery, an end effector) needs to be replaced. If a replaceable component is replaced the processor may return to gathering the usage of each of the components (Step 220). Further, a sensor (e.g., one of the sensors 20), the processor 22, or a component (e.g., components 110-180) may provide an audible signature indicating that the surgical instrument 10 is beyond its usage limit and must be replaced. The audible signature may be a default tone or tones, which may be specific to the component that is beyond its usage limit.

Additionally or alternatively, the processing unit 60 may receive data from the processor 22 gathered during Step 220 and compare the actual use of each of the components of the surgical instrument 10 with the usage limit of each of the components and transmit a signature to processor 22 to permit use of the surgical instrument 10. It is also within the scope of this disclosure that the processing unit 60 directly receives a signature from one or more components of the surgical instrument 10 (e.g., components 110-180).

In aspects of the present disclosure, the transmitter 24 sends the data to the cloud 55. The manufacturer of the surgical instrument 10 may be able to access the data in the cloud 55. The manufacturer of the surgical instrument 10 may use the data to determine if any components (e.g., components 110-180) are failing prematurely (i.e., a component reaching a respective usage limit before anticipated). If one or more components are failing prematurely, the manufacturer may utilize the data to improve the prematurely failing components in future revisions of the surgical instrument 10, to warn other customers of the premature failure, or to remove other surgical instruments 10 from use before the other surgical instruments 10 are used on patients.

In other aspects of the present disclosure, the actual usage of the components of the surgical instrument 10 may be used by the processing unit 60 to determine supply requirements (e.g., staple cartridges, clip cartridges, end effectors, etc.) for a facility (e.g., hospital, clinic, hospital network). The processing unit 60 may transmit the supply requirements via on-screen display, an automatic supply order, an email, and/or a text.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A powered handle for a surgical instrument, the handle comprising:
   a processor; and
   a component having a usage limit, the component configured to transmit a signature to the processor when the component is in proximity to its usage limit, the signature configured to provide indicia directly to a user that the component is in proximity to its usage limit before the handle is used.
2. The handle of paragraph 1, wherein the component is at least one of a motor, a gear, or an electronic component.
3. The handle of paragraph 1, wherein the indicia is at least one of visual, audible, or haptic.
4. The handle of paragraph 1, wherein the signature is acoustic and configured to be audible to a user.
5. The handle of paragraph 1, wherein the signature is configured to provide indicia of remaining life of the component.
6. The handle of paragraph 5 further comprising a display, the display configured to provide indicia of the remaining life of the component.
7. The handle of paragraph 1 further comprising a display, the display configured to provide indicia when the component is past its usage limit.
8. The handle of paragraph 1 further comprising a sensor associated with a second component and configured to detect when the second component is in proximity to its usage limit.
9. The handle of paragraph 8, wherein the sensor is configured to transmit a second signature to the processor when the second component is in proximity to its usage limit, the second signature configured to provide indicia directly to a user that the second component is in proximity to its usage limit before the handle is used.
10. A powered surgical instrument comprising:
   an end effector;
   a handle operatively associated with an end effector, the handle including a processor; and
   a component having a usage limit, the component configured to transmit a signature to the processor when the component is in proximity to its usage limit, the signature configured to provide indicia directly to a user that the component is in proximity to its usage limit before the handle is used.
11. The surgical instrument of paragraph 10, wherein the component is disposed within the end effector.
12. The surgical instrument of paragraph 10 further comprising a sensor associated with a second component, the sensor configured to detect when the second component is in proximity to its usage limit, the sensor is configured to transmit a second signature to the processor when the second component is in proximity to its usage limit, the second signature configured to provide indicia directly to a user that the second component is in proximity to its usage limit before the handle is used.
13. A method for using a surgical instrument, the method comprising:
   gathering usage of a component of a surgical instrument, the surgical instrument including:
      a processor;
      the component having a usage limit; and
      a handle operatively associated with an end effector;
   comparing the usage of the component with the usage limit of the component to determine when the component is in proximity to its usage limit; and
   transmitting a signature to the processor when the component is in proximity to its usage limit, the signature providing indicia directly to a user that the component is in proximity to its usage limit.
14. The method of paragraph 13, wherein the component transmits the signature.
15. The method of paragraph 13, wherein gathering usage of the component includes a sensor detecting the usage of the component and wherein the sensor transmits the signature.
16. The method of paragraph 13 further comprising disabling the surgical instrument when the component is past its usage limit.
17. The method of paragraph 13 further comprising transmitting data including the usage of the component with a transmitter disposed within the surgical instrument to a processing unit remote to the surgical instrument, the processing unit comparing the usage of the component with a usage limit of the component to determine when the component is in proximity to its usage limit and providing indicia to a user when the component is in proximity to its usage limit via the handle.
18. The method of paragraph 13 further comprising transmitting data including the usage of the component to a cloud with a transmitter disposed within the surgical instrument and analyzing the data at a location remote to the surgical instrument.
19. The method of paragraph 18 further comprising accessing the data from the cloud and determining supply needs of the surgical instrument from the data.
20. The method of paragraph 13 further comprising displaying a remaining life of the component before the using the surgical instrument.

## Claims

1. A powered handle for a surgical instrument, the handle comprising:
a processor; and
a component having a usage limit, the component configured to transmit a signature to the processor when the component is in proximity to its usage limit, the signature configured to provide indicia directly to a user that the component is in proximity to its usage limit before the handle is used.

2. The handle of claim 1, wherein the component is at least one of a motor, a gear, or an electronic component; and/or wherein the indicia is at least one of visual, audible, or haptic.

3. The handle of claim 1 or claim 2, wherein the signature is acoustic and configured to be audible to a user.

4. The handle of any preceding claim, wherein the signature is configured to provide indicia of remaining life of the component; preferably further comprising a display, the display configured to provide indicia of the remaining life of the component.

5. The handle of any preceding claim further comprising a display, the display configured to provide indicia when the component is past its usage limit.

6. The handle of any preceding claim further comprising a sensor associated with a second component and configured to detect when the second component is in proximity to its usage limit; preferably wherein the sensor is configured to transmit a second signature to the processor when the second component is in proximity to its usage limit, the second signature configured to provide indicia directly to a user that the second component is in proximity to its usage limit before the handle is used.

7. A powered surgical instrument comprising:
an end effector;
a handle operatively associated with an end effector, the handle including a processor; and
a component having a usage limit, the component configured to transmit a signature to the processor when the component is in proximity to its usage limit, the signature configured to provide indicia directly to a user that the component is in proximity to its usage limit before the handle is used.

8. The surgical instrument of claim 7, wherein the component is disposed within the end effector.

9. The surgical instrument of claim 7 or claim 8 further comprising a sensor associated with a second component, the sensor configured to detect when the second component is in proximity to its usage limit, the sensor is configured to transmit a second signature to the processor when the second component is in proximity to its usage limit, the second signature configured to provide indicia directly to a user that the second component is in proximity to its usage limit before the handle is used.

10. A method for using a surgical instrument, the method comprising:
gathering usage of a component of a surgical instrument, the surgical instrument including:
a processor;
the component having a usage limit; and
a handle operatively associated with an end effector;
comparing the usage of the component with the usage limit of the component to determine when the component is in proximity to its usage limit; and
transmitting a signature to the processor when the component is in proximity to its usage limit, the signature providing indicia directly to a user that the component is in proximity to its usage limit.

11. The method of claim 10, wherein the component transmits the signature.

12. The method of claim 10 or claim 11, wherein gathering usage of the component includes a sensor detecting the usage of the component and wherein the sensor transmits the signature; preferably further comprising disabling the surgical instrument when the component is past its usage limit.

13. The method of any of claims 10 to 12 further comprising transmitting data including the usage of the component with a transmitter disposed within the surgical instrument to a processing unit remote to the surgical instrument, the processing unit comparing the usage of the component with a usage limit of the component to determine when the component is in proximity to its usage limit and providing indicia to a user when the component is in proximity to its usage limit via the handle.

14. The method of any of claims 10 to 13 further comprising transmitting data including the usage of the component to a cloud with a transmitter disposed within the surgical instrument and analyzing the data at a location remote to the surgical instrument; preferably further comprising accessing the data from the cloud and determining supply needs of the surgical instrument from the data.

15. The method of any of claims 10 to 14 further comprising displaying a remaining life of the component before the using the surgical instrument.
